# EUROPEAN PATENT APPLICATION

(11) **EP 4 818 150 A1**
(43) Date of publication of application: **07.10.2026**
(21) Application number: 24897659.9
(22) Date of filing: 28.11.2024
(51) Int. Cl.: C07C 227/04, B01J 31/28, B01J 31/36, C07B 61/00, C07C 209/36, C07C 211/49, C07C 213/02, C07C 219/32, C07C 221/00, C07C 225/22, C07C 227/06, C07C 229/40, C07C 229/42, C07C 229/44, C07C 229/60, C07C 253/30, C07C 255/58, C07C 271/22, C07D 311/74

(54) **METHOD FOR PRODUCING AROMATIC AMINO COMPOUND**

(30) Priority: 30.11.2023 JP 2023202678; 29.02.2024 JP 2024029919
(71) Applicant: Nissan Chemical Corporation, Tokyo 103-6119 (JP)
(72) Inventor: NAGAO, Masato, Funabashi-shi, Chiba 274-8507 (JP)
(74) Representative: Wächtershäuser & Hartz Patentanwaltspartnerschaft mbB
(86) International application number: PCT/JP2024/042210
(87) International publication number: WO 2025/115975

(57) **Abstract**

Provided is a novel method for producing an aromatic amino compound having at least one multiple bond selected from a carbon-carbon bond, a carbon-nitrogen bond, and a carbon-oxygen bond in a moiety other than the aromatic ring, and being useful as an intermediate for producing agrochemicals, pharmaceuticals, and electronic materials.

The method for producing an aromatic amino group includes catalytic reduction of an aromatic nitro compound having, in a moiety other than the aromatic ring, at least one multiple bond selected from a carbon-carbon bond, a carbon-nitrogen bond, and a carbon-oxygen bond, over a Pt/C catalyst in the presence of at least one phosphorus compound (A) selected from the group consisting of a phosphorus compound represented by the following formula (1), a phosphorus compound represented by the following formula (2), and a phosphorus compound represented by the following formula (3).

P(R¹)₃ (1)

PO(R²)₃ (2)

(R³)₂P-R⁴-P(R³)₂ (3)

The definitions of the symbols in the formulas (1) to (3) are as defined in the specification.

## Description

### TECHNICAL FIELD

The present invention relates to a method for producing an aromatic amino compound having at least one multiple bond selected from a carbon-carbon bond, a carbon-nitrogen bond, and a carbon-oxygen bond in a moiety other than the aromatic ring, and being useful as an intermediate for producing agrochemicals, pharmaceuticals, and electronic materials.

### BACKGROUND ART

It is known that aromatic amino compounds can be produced by reducing aromatic nitro compounds. However, when an aromatic nitro compound having at least one multiple bond selected from a carbon-carbon bond, a carbon-nitrogen bond, and a carbon-oxygen bond in a moiety other than the aromatic ring is reduced to the corresponding amino compound, the multiple bond may also be reduced depending on the conditions. It is thus generally difficult to reduce only the nitro group to an amino group while retaining the multiple bond, and methods have been sought for selectively reducing only the nitro group of aromatic nitro compounds to an amino group.

As a document describing such a reduction reaction, for example, Patent Document 1 discloses a method for producing a substituted aromatic amino compound having at least one carbon-carbon, carbon-nitrogen or carbon-oxygen bond in its aromatic moiety or side chain, by catalytic hydrogenation of the corresponding substituted aromatic nitro compound in the presence of a modified noble metal catalyst, wherein the noble metal catalyst is rhodium, ruthenium, iridium, platinum, or palladium, modified with an inorganic or organic phosphorus compound and having an oxidation state of less than 5.

Furthermore, Patent Document 2 proposes a method for selectively hydrogenating an aromatic nitro compound to an aromatic amino compound in the presence of a Pt/C catalyst poisoned with a trace amount of iron.

### PRIOR ART DOCUMENTS

### PATENT DOCUMENTS

Patent Document 1: JP-A-2001-501201
Patent Document 2: DE-A-102011003590

### DISCLOSURE OF INVENTION

### TECHNICAL PROBLEM

In this way, methods for selectively reducing only the nitro group of aromatic nitro compounds to an amino group have been proposed. However, there has been a demand for a method capable of reducing only the nitro group of aromatic nitro compounds to an amino group with higher selectivity and without compromising the reaction rate.

It is accordingly an object of the present invention to provide a novel method for producing an aromatic amino compound useful as an intermediate for producing agrochemicals, pharmaceuticals, and electronic materials, by reducing the nitro group of an aromatic nitro compound having at least one multiple bond selected from a carbon-carbon bond, a carbon-nitrogen bond, and a carbon-oxygen bond in a moiety other than the aromatic ring to an amino group with higher selectivity and without compromising the reaction rate.

### SOLUTION TO PROBLEM

In view of the foregoing, the present inventors have conducted intensive studies and have found a method for selectively reducing only the nitro group of an aromatic nitro compound having, in a moiety other than the aromatic ring, at least one multiple bond selected from a carbon-carbon bond, a carbon-nitrogen bond, and a carbon-oxygen bond by catalytic reduction over a supported catalyst of specific noble metal in the presence of a specific phosphorus compound. The present invention has been accomplished based on such a finding.

That is, the present invention provides the following solutions to the above-described problems.
[1] A method for producing an aromatic amino compound, comprising: catalytic reduction of an aromatic nitro compound having, in a moiety other than an aromatic ring thereof, at least one multiple bond selected from the group consisting of a carbon-carbon bond, a carbon-nitrogen bond, and a carbon-oxygen bond, over a Pt/C catalyst in the presence of at least one phosphorus compound (A) selected from the group consisting of a phosphorus compound represented by the following formula (1), a phosphorus compound represented by the following formula (2), and a phosphorus compound represented by the following formula (3):

   P(R¹)₃ (1)

   where a plurality of R¹ each independently represent a hydrogen atom, a C₁₋₁₈ alkyl group, a C₁₋₁₈ alkenyl group, a C₁₋₁₈ alkoxy group, a C₆₋₂₄ aryl group, a C₆₋₂₄ aryloxy group, a C₃₋₁₈ cycloalkyl group, or a C₃₋₁₈ cycloalkoxy group, each of which may be substituted;

   PO(R²)₃ (2)

   where a plurality of R² each independently represent a hydrogen atom, a C₁₋₁₈ alkyl group, a C₁₋₁₈ alkenyl group, a C₁₋₁₈ alkoxy group, a C₆₋₂₄ aryl group, a C₆₋₂₄ aryloxy group, a C₃₋₁₈ cycloalkyl group, or a C₃₋₁₈ cycloalkoxy group, each of which may be substituted; and

   (R³)₂P-R⁴-P(R³)₂ (3)

   where a plurality of R³ each independently represents a C₁₋₁₈ alkyl group, a C₁₋₁₈ alkenyl group, a C₁₋₁₈ alkoxy group, a C₆₋₂₄ aryl group, a C₆₋₂₄ aryloxy group, a C₃₋₁₈ cycloalkyl group, or a C₃₋₁₈ cycloalkoxy group, each of which may be substituted; and R⁴ represents a C₁₋₁₈ alkylene group, a C₁₋₁₈ alkenylene group, a C₁₋₁₈ alkylenedioxy group, a C₆₋₂₄ arylene group, a C₆₋₂₄ arylenedioxy group, a C₃₋₁₈ cycloalkylene group, or a C₃₋₁₈ cycloalkylenedioxy group, each of which may be substituted.
[2] The method for producing an aromatic amino compound according to [1], wherein the phosphorus compound (A) is P(OMe)₃ or P(OEt)₃.
[3] The method for producing an aromatic amino compound according to [1] or [2], wherein the phosphorus compound (A) is used in an amount of 5 to 50 mol% relative to the aromatic amino compound.
[4] The method for producing an aromatic amino compound according to any one of [1] to [3], wherein the aromatic nitro compound has any one of structures represented by the following formulas (1) to (3): 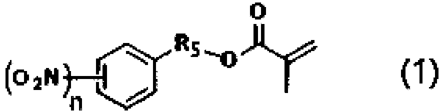 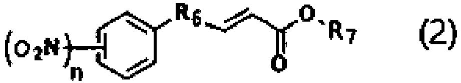 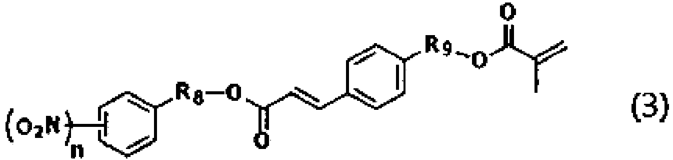 where R₅, R₆, R₈, and R₉ each independently represent a single bond or a divalent group; R₇ represents a hydrogen atom or a monovalent group; and n represents an integer of 1 or 2.
[5] The method for producing an aromatic amino compound according to any one of [1] to [4], wherein the Pt/C catalyst comprises 0.5 to 5 wt% platinum based on the total weight of the Pt/C catalyst.
[6] The method for producing an aromatic amino compound according to any one of [1] to [5], wherein the Pt/C catalyst is poisoned with iron.
[7] The method for producing an aromatic amino compound according to any one of [1] to [6], wherein the Pt/C catalyst is used in an amount of 1 to 20 wt% relative to the aromatic nitro compound.
[8] The method for producing an aromatic amino compound according to any one of [1] to [7], wherein the catalytic reduction is carried out at a reaction temperature of 0 to 100°C for a reaction time of 0.5 to 20 hours.
[9] The method for producing an aromatic amino compound according to any one of [1] to [8], wherein the catalytic reduction is carried out in the further presence of a vanadium compound.
[10] The method for producing an aromatic amino compound according to [9], wherein the vanadium compound is VO(acac)₂.
[11] The method for producing an aromatic amino compound according to [9] or [10], wherein the vanadium compound is used in an amount of 0.1 to 1.0 mol% relative to the aromatic nitro compound.

### ADVANTAGEOUS EFFECTS OF INVENTION

According to the method of the present invention, only the nitro group of an aromatic nitro compound having, in a moiety other than the aromatic ring, at least one multiple bond selected from a carbon-carbon bond, a carbon-nitrogen bond, and a carbon-oxygen bond (hereinafter also simply referred to as a "compound (DN)") can be selectively reduced without compromising the reaction rate. It is therefore possible to efficiently and cost-effectively produce an aromatic amino compound having, in a moiety other than the aromatic ring, at least one multiple bond selected from a carbon-carbon bond, a carbon-nitrogen bond, and a carbon-oxygen bond, and being useful as an intermediate for producing agrochemicals, pharmaceuticals, and electronic materials.

### DESCRIPTION OF EMBODIMENTS

Throughout the present specification, the following terms and abbreviations have the following meanings.

A numerical range expressed using "to" means a range including numerical values described before and after "to" as the lower and upper limits. In a series of numerical ranges described in the present specification, the upper limit or lower limit of a numerical range may be replaced by the upper limit or lower limit of another numerical range in the same series. The upper limit or lower limit of any numerical range described in the present specification may be replaced by any of numerical values indicated in Examples.

In the present specification, Me represents a methyl group, Et represents an ethyl group, Pr represents a propyl group, Bu represents a butyl group, n- represents normal, t- represents tertiary, o- represents ortho, Cy represents a cyclohexyl group, Ph represents a phenyl group, Bn represents a benzyl group, Tol represents a tolyl group, and acac represents acetylacetonate.

Hereinafter, the method of the present invention for producing an aromatic amino compound will be described.

Although the details of the method of the present invention for producing an aromatic amino compound will be described below with reference to specific embodiments, the present invention is not limited to these embodiments, and various changes and modifications can be made without departing from the scope of the present invention.

As the method of the present invention, a method for producing an aromatic amino compound as represented in the following Reaction Scheme 1 may be exemplified.

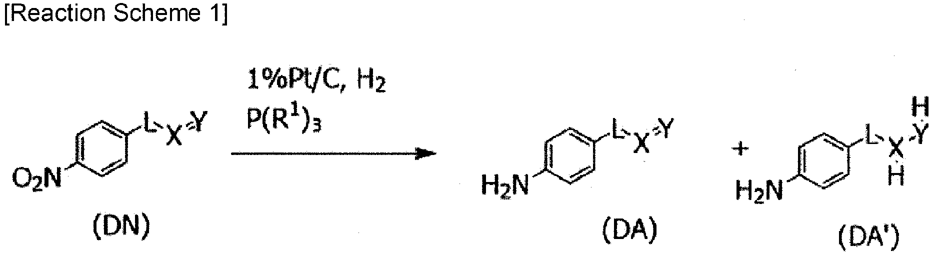

In the above scheme, L represents a single bond or a divalent group; X represents CR¹¹; Y represents O, NR¹², or CR¹³R¹⁴; R¹¹, R¹², R¹³, and R¹⁴ each independently represent a hydrogen atom or a monovalent group; when R¹¹, R¹², R¹³, and R¹⁴ are not bonded, the bond between X and Y may be a triple bond; and a plurality of R¹ are each independently as defined above.

When the bond between X and Y is a double bond, X and Y may together form a ring structure such as a five-membered ring or a six-membered ring. Specific examples of the ring structure include cyclic olefins such as cyclohexene, cyclohexadiene, pyran, dihydropyran, dihydrofuran, cyclohexen-1-one, dihydropyrrole, dihydropyridine, and tetrahydropyridine.

In other words, when the compound (DN), an aromatic nitro compound, has, for example, cyclohexene as the ring structure, it has the following structure.

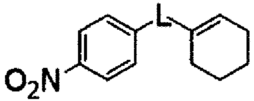

In the above formula, L represents a single bond or a divalent group.

Furthermore, when the bond between X and Y is a double bond, Y may be bonded to the nitro-substituted benzene ring. As specific examples, compounds having the following structure may be mentioned.

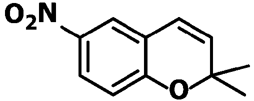

The present invention is the method for producing an aromatic amino compound by catalytic reduction of an aromatic nitro compound having, in a moiety other than the aromatic ring, at least one multiple bond selected from a carbon-carbon bond, a carbon-nitrogen bond, and a carbon-oxygen bond, over a Pt/C catalyst in the presence of at least one phosphorus compound (A) selected from the group consisting of a phosphorus compound represented by the following formula (1), a phosphorus compound represented by the following formula (2), and a phosphorus compound represented by the following formula (3).

The aromatic nitro compound used in the present invention has, in a moiety other than the aromatic ring, at least one multiple bond selected from a carbon-carbon bond, a carbon-nitrogen bond, and a carbon-oxygen bond.

The aromatic nitro compound is preferably an aromatic mononitro compound or an aromatic dinitro compound.

Among the above multiple bonds, an α,β-unsaturated carbonyl group is preferred. Examples of the aromatic nitro compound having an α,β-unsaturated carbonyl group as the multiple bond include those having structures represented by the following formulas (I) to (III).

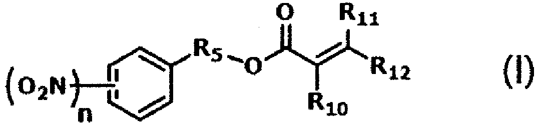

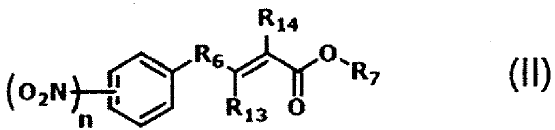

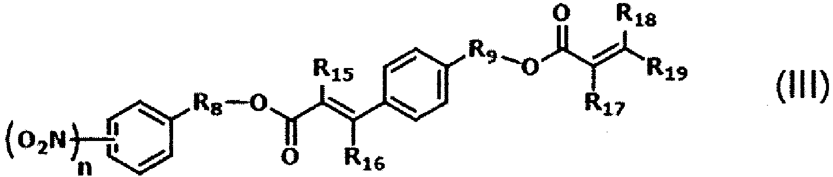

In the above formulas, R₅, R₆, R₈, and R₉ each independently represent a single bond or a divalent group; R₇ represents a hydrogen atom or a monovalent group; R₁₀, R₁₁, R₁₂, R₁₃, R₁₄, R₁₅, R₁₆, R₁₇, R₁₈, and R₁₉ each independently represent a hydrogen atom or a methyl group; and n represents an integer of 1 or 2.

As the compounds represented by the formulas (I) to (III), the following compounds (1) to (3) are preferred.

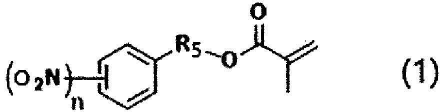

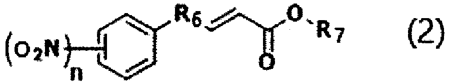

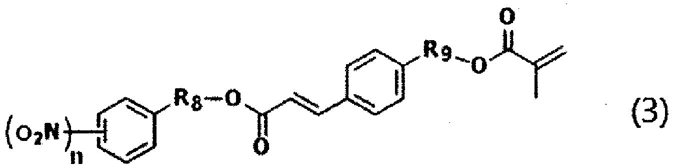

In the above formulas, R₅, R₆, R₈, and R₉ each independently represent a single bond or a divalent group; R₇ represents a hydrogen atom or a monovalent atom; and n represents an integer of 1 or 2.

Examples of the divalent group for R₅, R₆, R₈, and R₉ include C₁₋₂₀ alkylene groups which may be unsubstituted or substituted with fluorine. In the C₁₋₂₀ alkylene groups, -CH₂- or -CF₂- may be replaced with a group selected from the group consisting of -O-, -COO-, -OCO-, -NHCO-, -CONH-, -NH-, a divalent carbocyclic ring, and a divalent heterocyclic ring, provided that the groups selected therefrom are not adjacent to each other. Among these, an unsubstituted C₁₋₆ alkylene group is preferred in which -CH₂- on the side bonded to the benzene ring may be replaced with a group selected from -O-, -COO-, -OCO-, -NHCO-, -CONH-, and -NH-, provided that the groups selected therefrom are not adjacent to each other.

Examples of the monovalent group for R₇ include C₁₋₂₀ alkyl groups, which may be unsubstituted or substituted with fluorine. In the C₁₋₂₀ alkyl groups, -CH₂- or -CF₂- may be replaced with a group selected from the group consisting of -O-, -COO-, -OCO-, - NHCO-, -CONH-, -NH-, a divalent carbocyclic ring, and a heterocyclic ring, provided that the groups selected therefrom are not adjacent to each other. Among these, an unsubstituted C₁₋₆ alkyl group is preferred in which -CH₂- may be replaced with a group selected from -O-, -COO-, -OCO-, -NHCO-, -CONH-, and -NH-, provided the groups selected therefrom are not adjacent to each other.

The phosphorus compound used in the present invention is at least one phosphorus compound (A) selected from the group consisting of phosphorus compounds represented by the following formulas (1), (2) and (3).

P(R¹)₃ (1)

In the formula (1), a plurality of R¹ each independently represent a hydrogen atom, a C₁₋₁₈ alkyl group, a C₁₋₁₈ alkenyl group, a C₁₋₁₈ alkoxy group, a C₆₋₂₄ aryl group, a C₆₋₂₄ aryloxy group, a C₃₋₁₈ cycloalkyl group, or a C₃₋₁₈ cycloalkoxy group, each of which may be substituted.

PO(R²)₃ (2)

In the formula (2), a plurality of R² each independently represent a hydrogen atom, a C₁₋₁₈ alkyl group, a C₁₋₁₈ alkenyl group, a C₁₋₁₈ alkoxy group, a C₆₋₂₄ aryl group, a C₆₋₂₄ aryloxy group, a C₃₋₁₈ cycloalkyl group, or a C₃₋₁₈ cycloalkoxy group, each of which may be substituted.

(R³)₂P-R⁴-P(R³)₂ (3)

In the formula (3), a plurality of R³ each independently represents a C₁₋₁₈ alkyl group, a C₁₋₁₈ alkenyl group, a C₁₋₁₈ alkoxy group, a C₆₋₂₄ aryl group, a C₆₋₂₄ aryloxy group, a C₃₋₁₈ cycloalkyl group, or a C₃₋₁₈ cycloalkoxy group, each of which may be substituted; and R⁴ represents a C₁₋₁₈ alkylene group, a C₁₋₁₈ alkenylene group, a C₁₋₁₈ alkylenedioxy group, a C₆₋₂₄ arylene group, a C₆₋₂₄ arylenedioxy group, a C₃₋₁₈ cycloalkylene group, or a C₃₋₁₈ cycloalkylenedioxy group, each of which may be substituted.

Specific examples of R¹ in the formula (1) include methyl, ethyl, normal propyl (*n-*Pr), isopropyl (*i*-Pr), normal butyl (*n*-Bu), tertiary butyl (*t*-Bu), phenyl (Ph), benzyl (Bn), methoxy (OMe), ethoxy (OEt), normal propoxy (O*n*-Pr), normal butoxy (O*n*-Bu), phenoxy (OPh), and benzyloxy. Among others, *n*-Bu, Ph, OMe, OEt, O*n*-Bu, and OPh are preferred. From the viewpoint of higher selectivity, OMe and OEt are more preferred.

Specific examples of R² in the formula (2) include hydrogen, methyl, ethyl, *n*-Pr, *i-*Pr, *n*-Bu, *t*-Bu, *n*-octyl, cyclohexyl, Ph, *p*-methylphenyl, *p*-methoxyphenyl, benzyl, OMe, OEt, normal propoxy, normal butoxy, OPh, and benzyloxy. From the viewpoint of higher selectivity, hydrogen, methyl, *n*-Bu, *n*-octyl, and Ph are preferred, and hydrogen, methyl, and Ph are more preferred.

Specific examples of R³ in the formula (3) include hydrogen, methyl, ethyl, *n*-Pr, *i-*Pr, *n*-Bu, *t*-Bu, *n*-octyl, cyclohexyl, Ph, *p*-methylphenyl, *p*-methoxyphenyl, benzyl, OMe, OEt, normal propoxy, normal butoxy, OPh, and benzyloxy. From the viewpoint of higher selectivity, methyl, ethyl, OMe, OEt, and Ph are preferred. From the viewpoint of even higher selectivity, methyl, OMe, OEt, and Ph are more preferred.

Specific examples of R⁴ in the formula (3) include methylene, ethylene, propylene, butylene, pentylene, hexylene, 1,2-phenylene, 1,3-phenylene, 2,2'-biphenylene, 2,2'-binaphthylene, 1,8-naphthylene, 1,8-(9,9-dimethyl)xanthenylene, and 1,1'-ferrocenylene. Among others, methylene, ethylene, propylene, butylene, 1,2-phenylene, 2,2'-biphenylene, 2,2'-binaphthylene, 1,8-naphthylene, and 1,1'-ferrocenylene are preferred. From the viewpoint of higher selectivity, methylene, ethylene, propylene, butylene, and 2,2'-binaphthylene are more preferred.

As examples of substituents that R¹, R² and R³ in the above formulas may have, there may be mentioned a methyl group, an ethyl group, an *i*-propyl group, a *t*-butyl group, a phenyl group, a hydroxy group, a methoxy group, an *i*-propoxy group, a *t-*butoxy group, an amino group, a dimethylamino group, a cyano group, a formyl group, a carboxy group, a sulfoxy group, a fluoro group, a chloro group, a bromo group, and an iodo group.

Specific examples of the phosphorus compound (A) include PMe₃, PEt₃, P(*n*-Pr)₃, P(*n*-Bu)₃, P(*t*-Bu)₃, P(*n*-C₆H₁₃)₃, P(*n*-C₈H₁₇)₃, PCy₃, PBn₃, PPh₃, P(*o*-Tol)₃, P(OMe)₃, P(OEt)₃, P(O*n*-Pr)₃, P(O*n*-Bu)₃, P(OPh)₃, P(OBn)₃, P(O)(*n*-Bu)₃, P(O)Ph₃, (±)-BINAP (2,2'-bis(diphenylphosphino)-1,1-binaphthyl), (+)-BINAP, (-)-BINAP, depe (1,2-bis(diethylphosphino)ethane), dppm (1,1-bis(diphenylphosphino)methane), dppe (1,2-bis(diphenylphosphino)ethane), dppp (1,3-bis(diphenylphosphino)propane), dppb (1,4-bis(diphenylphosphino)butane), dppbz (1,2-bis(diphenylphosphino)benzene), dppf (1,1'-bis(diphenylphosphino)ferrocene), and Xantphos.

Among others, P(*n*-Bu)₃, PPh₃, P(OMe)₃, P(OEt)₃, P(O*n*-Bu)₃, and P(OPh)₃ are preferred. From the viewpoint of higher selectivity, P(OMe)₃ and P(OEt)₃ are more preferred.

The amount of the phosphorus compound (A) used in the present invention is preferably 50 mol% or less, more preferably 35 mol% or less, relative to the compound (DN), an aromatic nitro compound, from the viewpoint of ease of removal after the reaction. From the viewpoint of higher selectivity, the amount of the phosphorus compound (A) used is preferably 5 mol% or more, more preferably 10 mol% or more.

The amount of the Pt/C catalyst used in the reaction relative to the compound (DN), an aromatic nitro compound, is preferably 1 wt% or more, more preferably 3 wt% or more, and is preferably 20 wt% or less, more preferably 10 wt% or less.

The amount of platinum supported in the Pt/C catalyst is preferably 0.5 to 5 wt%, more preferably 1 to 5 wt%, based on the total weight of the Pt/C catalyst.

In the reaction, a Pt/C catalyst poisoned with S, Cu, V, or Fe is preferably used, and a Pt/C catalyst poisoned with Fe is more preferably used. From the viewpoint of suppressing side reactions, the amount of Fe used for poisoning the Pt/C catalyst is preferably 0.05 wt% or more, more preferably 0.1 wt% or more, based on the total weight of the Fe-poisoned Pt/C catalyst. From the viewpoint of preventing a decrease in catalytic activity, the amount of Fe used for poisoning is preferably 1.0 wt% or less, more preferably 0.5 wt% or less.

From the viewpoint of suppressing the formation of azoxy compounds, it is preferable to carry out the reaction in the further presence of a vanadium compound. As such a vanadium compound, VO(acac)₃, VO(acac)₂, NH₄VO₃, V₂O₅, VOCl₃, VCl₆, [VO(SCN)₄]²⁻, VOSO₄, LiVO₃, NaVO₃, KVO₃, and VCl₃ are preferred, and VO(acac)₂ is more preferred.

The vanadium compound, when used in an excessive amount in the reaction, is likely to remain in the resulting product. Thus, the amount of the vanadium compound used is preferably 1.0 mol% or less, more preferably 0.2 mol% or less, relative to the compound (DN). From the viewpoint of sufficiently suppressing the formation of azoxy compounds, the amount of the vanadium compound used is preferably 0.1 mol% or more.

The reaction may be carried out in a solvent. As such a reaction solvent, any solvent can be used as long as it is stable under the reaction conditions, inert, and does not interfere with the reaction. Examples of the reaction solvent include water, alcohols, amines, aprotic polar solvents (such as DMF (dimethylformamide), DMSO (dimethyl sulfoxide), DMAc (dimethylacetamide), and NMP (N-methylpyrrolidone)), ethers (such as Et₂O, *i*-Pr₂O, TBME (tert-butyl methyl ether), CPME (cyclopentyl methyl ether), THF (tetrahydrofuran), and dioxane), aliphatic hydrocarbons (such as pentane, hexane, heptane, and petroleum ether), aromatic hydrocarbons (such as benzene, toluene, xylene, mesitylene, chlorobenzene, dichlorobenzene, nitrobenzene, and tetralin), halogenated hydrocarbons (such as chloroform, dichloromethane, tetrachloromethane, and dichloroethane), lower fatty acid esters (such as methyl acetate, ethyl acetate, butyl acetate, and methyl propionate), and nitriles (such as acetonitrile, propionitrile, and butyronitrile), and the like. These solvents can be appropriately selected in view of ease of the reaction, and may be used alone or as a mixture of two or more thereof. In some cases, the above solvents may be used in an anhydrous state by treatment with an appropriate dehydrating or drying agent.

The reaction temperature for the catalytic reduction is usually from -90°C to 200°C, and is preferably from 0°C to 100°C.

The reaction time for the catalytic reduction is usually from 0.05 hours to 100 hours, and is preferably from 0.5 hours to 20 hours, more preferably from 0.5 hours to 10 hours.

The reaction pressure for the catalytic reaction is usually from atmospheric pressure to 10 MPaG, and is preferably from atmospheric pressure to 0.8 MPaG.

### EXAMPLES

The present invention will now be described in further detail with reference to Examples. It should, however, be understood that the present invention is not limited to these Examples. The analytical devices and analytical conditions employed in the Examples are as follows.
¹H-NMR
   Device: ECZ (400 MHz), manufactured by JEOL Ltd.
   Measurement solvent: CDCl₃
   Reference: tetramethylsilane (TMS) (δ = 0.0 ppm for ¹H of TMS)
HPLC
   Device: HPLC with LC-20AD, manufactured by Shimadzu Corporation
   Column: Inertsil ODS-3 (GL Science), 5 µm, Φ 4.6 × 250 mm
   Column temperature: 40°C
   Eluent: (A) acetonitrile/(B) 50 mM potassium phosphate buffer (pH=7)
   A/B = 35/65 (0 to 10 min.) -60/40 (13 to 30 min.) (v/v)
   Flow rate: 1.0 mL/min.
   Detection method: UV (254 nm)
   Data acquisition time: 30 min.

### [Synthesis Examples of Aromatic Amino Compounds]

### <Synthesis Example 1> Synthesis of 2-(3,5-diaminobenzoyloxy)ethylmethacrylate (compound DA-1)

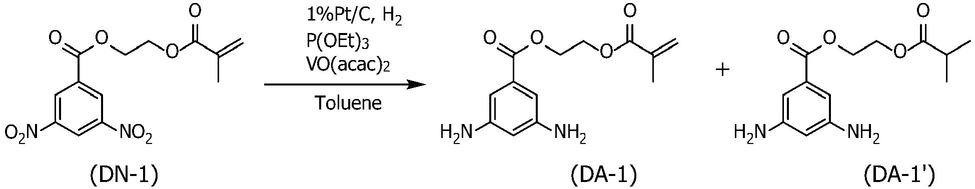

Nitro compounds used herein are known compounds and can be synthetized according to known methods described in the literature. For example, 2-(3,5-dinitrobenzoyloxy)ethyl methacrylate can be obtained by reaction in accordance with the method described in Japanese Patent No. 5560715.

### (Synthesis Example 1-a-1)

To a 200-mL pressure vessel were added 2-(3,5-dinitrobenzoyloxy)ethyl methacrylate (5.00 g, 15.4 mmol), toluene (50.0 g), triethyl phosphite (0.512 g, 3.08 mmol), 1 wt% Pt/C catalyst poisoned with 0.2 wt% iron (55.0 wt% water-containing type, 0.556 g), and bis(acetylacetonato)oxovanadium (8.94 mg, 30.8 µmmol). The resulting mixture was stirred at 40°C for 4 hours under a hydrogen atmosphere at a gauge pressure of 0.60 MPa.

Upon HPLC analysis of the reaction mixture, it was found that the HPLC relative area percentage of DA-1 was 98.8%, the reaction yield was 100%, and no over-reduced product (DA-1') was detected.

After completion of the reaction, ethyl acetate (20.0 g) was added to the reaction mixture, and the catalyst was removed by filtration. The residue on the filter was washed twice with ethyl acetate (5.00 g each). Water (20.0 g) was added to the filtrate, the resulting mixture was stirred, and the aqueous layer was separated. This operation was repeated twice. The organic layer was then concentrated to 41.2 g, followed by addition of toluene (50.0 g) and reconcentration to 41.2 g. The concentrate was cooled to 0°C. The crystalline precipitate was collected by filtration, washed twice with toluene (15.0 g each) and twice with water (5.0 g each), and dried under reduced pressure at 40°C, thereby obtaining a crystalline powder (DA-1) (3.44 g, 86.5% yield).

Upon HPLC analysis of the obtained crystalline powder, it was found that the HPLC relative area percentage of DA-1 was 100.0%, and no over-reduced product (DA-1') was detected.

¹H-NMR (CDCl₃): δ 6.77 (d, J = 2.4Hz, 2H, Ar), 6.19 (dd, J = 2.4Hz, 2.2Hz, 1H, =CH), 6.14 (br, 1H, Ar), 5.59 (dd, J = 2.4Hz, 2.2Hz, 1H,=CH), 4.48 (m, 4H, CH₂CH₂), 3.59 (br, 4H, NH₂), 1.95 (s, 3H, Me).

### (Comparative Synthesis Example)

To a 200-mL pressure vessel were added 2-(3,5-dinitrobenzoyloxy)ethyl methacrylate (2.00 g, 6.17 mmol), toluene (20.0 g), a 50 wt% aqueous solution of phosphinic acid (0.163 g, 1.23 mmol), and 1 wt% Pt/C catalyst poisoned with 0.2 wt% iron (55.0 wt% water-containing type, 0.444 g). The resulting mixture was stirred at 40°C under a hydrogen atmosphere at a gauge pressure of 0.60MPa. Upon HPLC analysis of the reaction mixture after 6 hours of stirring, it was found that the reaction had not yet reached completion. The LC relative area percentage of DA-1 was 42.3%, and the LC relative area percentage of the over-reduced product (DA-1') was 0.3%. The reaction mixture was further stirred. Upon HPLC analysis of the reaction mixture after a total of 24 hours of stirring, it was found that the HPLC relative area percentage of DA-1 was 96.5%, the reaction yield was 72%, and the HPLC relative area percentage of the over-reduced product (DA-1') was 1.0%.

Synthesis Examples 1-a-2 to 1-a-8 were carried out in accordance with the method described in Synthesis Example 1-a-1, except that the type of the phosphorus compound used as the additive, the amount of DN-1 used, the amount of 1 wt% Pt/C catalyst poisoned with 0.2 wt% iron, the amount of bis(acetylacetonato)oxovanadium (VO(acac)₂) added, and the amount of toluene used as the solvent were varied.

For each of Synthesis Examples 1-a-1 to 1-a-8 and Comparative Synthesis Example, the type of the additive, the amount of DN-1 used, the amount of 1 wt% Pt/C catalyst added, the amount of bis(acetylacetonato)oxovanadium (VO(acac)₂) added, the amounts of toluene as the solvent and of ethyl acetate, the LC relative area percentage of DA-1, the HPLC relative area percentage of the over-reduced product (DA-1'), the selectivity, and the reaction yield are summarized in Table 1.

The selectivity shown in the table was determined as follows. $Selectivity \left(%\right) = DA - 1 / \left(DA-1+DA-1′\right) \times 100$

**Table 1**

| Synthesis Example | Additive (0.2 eq.) | Amount of DN-1 used (g) | Amount of 1% Pt/C catalyst added (wt%) | Amount of VO(acac)₂ added (mol%) | Toluene (parts) | Ethyl acetate (parts) |
|---|---|---|---|---|---|---|
| 1-a-1 | P(OEt)₃ | 5.0 | 5 | 0.2 | 10 | 0 |
| 1-a-2 | P(OEt)₃ | 9.0 | 5 | 0.0 | 10 | 0 |
| 1-a-3 | P(OEt)₃ | 2.6 | 5 | 0.0 | 50 | 0 |
| 1-a-4 | P(OEt)₃ | 2.0 | 10 | 0.0 | 10 | 0 |
| 1-a-5 | P(OEt)₃ | 2.0 | 10 | 0.0 | 10 | 4 |
| 1-a-6 | P(OMe)₃ | 2.0 | 10 | 0.0 | 10 | 0 |
| 1-a-7 | P(OPh)₃ | 2.0 | 10 | 0.0 | 10 | 0 |
| 1-a-8 | P(n-Bu)₃ | 2.0 | 10 | 0.0 | 10 | 0 |
| Comparative Synthesis Example | 50% H₃PO₂ aq. | 2.0 | 10 | 0.0 | 10 | 0 |

**Table 1 (Continued)**

| Synthesis Example | Reaction time (hr) | HPLC relative area percentage (%) DA-1 | HPLC relative area percentage (%) DA-1' | Selectivity (%) | Reaction yield (%) |
|---|---|---|---|---|---|
| 1-a-1 | 4 | 98.8 | 0 | 100 | 100 |
| 1-a-2 | 7 | 98.7 | 0 | 100 | 96 |
| 1-a-3 | 4 | 99.4 | 0 | 100 | 96 |
| 1-a-4 | 2 | 99.6 | 0 | 100 | 100 |
| 1-a-5 | 2 | 99.6 | 0 | 100 | 100 |
| 1-a-6 | 4 | 100 | 0 | 100 | 96 |
| 1-a-7 | 4 | 97.1 | 2.9 | 97.1 | 95 |
| 1-a-8 | 4 | 90.9 | 1.5 | 98.4 | 70 |
| Comparative Synthesis Example | 6 | 42.3 | 0.3 | 99.2 | - |
| | 24 | 96.5 | 1.0 | 99.0 | 72 |

It was found from the results shown in Table 1 that, in Synthesis Examples 1-a-1 to 1-a-8 in which the catalytic reduction was carried out over a Pt/C catalyst in the presence of a phosphorus compound (A), only the nitro group of the aromatic nitro compound was reduced to an amino group with high selectivity in a short time, that is, without compromising the reaction rate.

### <Synthesis Example 2> Synthesis of (2-(2,4-diaminophenyl)ethyl) (E)-4-(6-(methacryloyloxy)hexyloxy)cinnamate (compound DA-2)

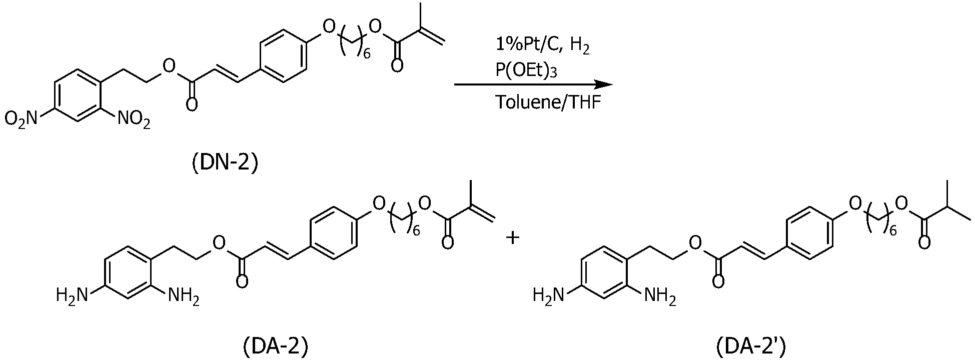

Nitro compounds used herein are known compounds and can be synthetized according to known methods described in the literature. For example, (2-(2,4-dinitrophenyl)ethyl) (E)-4-(6-(methacryloyloxy)hexyloxy)cinnamate can be obtained by reaction in accordance with the method described in Japanese Patent No. 6733552.

### (Synthesis Example 2)

To a 200-mL pressure vessel were added (2-(2,4-dinitrophenyl)ethyl) (E)-4-(6-(methacryloyloxy)hexyloxy)cinnamate (2.00 g, 3.80 mmol), toluene(10.0 g), THF(10.0 g), triethyl phosphite (0.200 g, 1.20 mmol), and 1 wt% Pt/C catalyst poisoned with 0.2 wt% iron (55.0 wt% water-containing type, 0.444 g). The resulting mixture was stirred at 40°C for 2 hours under a hydrogen atmosphere at a gauge pressure of 0.30 MPa.

Upon HPLC analysis of the reaction mixture, it was found that the HPLC relative area percentage of DA-2 was 97.8%, the reaction yield was 96%, and no over-reduced product (DA-2') was detected.

After completion of the reaction, the catalyst was removed by filtration, and the residue on the filter was washed twice with THF (2.00 g each). Water (20.0 g) was added to the filtrate, the resulting mixture was stirred, and the aqueous layer was separated. This operation was repeated twice. The organic layer was then concentrated to 10.0 g, followed by addition of toluene (20.0 g) and reconcentration to 10.0 g. The concentrate was cooled to 0°C. The crystalline precipitate was collected by filtration, washed twice with toluene (2.00 g each) and twice with water (2.00 g each), and dried under reduced pressure at 40°C, thereby obtaining a crystalline powder (DA-2) (1.40 g, 78.8% yield).

Upon HPLC analysis of the obtained crystalline powder, it was found that the HPLC relative area percentage of DA-2 was 98.2%, and no over-reduced product (DA-2') was detected.

### <Synthesis Example 3> Synthesis of ethyl (E)-4-aminocinnamate (compound DA-3)

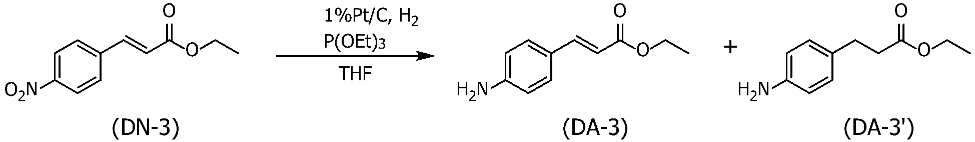

Ethyl (E)-4-Nitrocinnamate used herein can be a commercially available product, for example, from Tokyo Chemical Industry Co., Ltd.

### (Synthesis Example 3)

To a 200-mL pressure vessel were added ethyl (E)-4-nitrocinnamate (2.00 g, 9.04 mmol), THF (20.0 g), triethyl phosphite (0.200 g, 1.20 mmol), and 1 wt% Pt/C catalyst poisoned with 0.2 wt% iron (55.0 wt% water-containing type, 0.444 g). The resulting mixture was stirred at 40°C for 3 hours under a hydrogen atmosphere at a gauge pressure of 0.30 MPa.

Upon HPLC analysis of the reaction mixture, it was found that the LC relative area percentage of DA-3 was 99.5%, the reaction yield was 98%, and no over-reduced product (DA-3') was detected.

After completion of the reaction, the catalyst was removed by filtration, and the residue on the filter was washed twice with THF (2.00 g each). The filtrate was concentrated to 10.0 g, followed by addition of toluene (20.0 g) and reconcentration to 10.0 g. Heptane (10.0 g) was added to the concentrate. The resulting mixture was cooled to 0°C. The crystalline precipitate was collected by filtration, washed twice with heptane (2.00 g each), and dried under reduced pressure at 40°C, thereby obtaining a crystalline powder (DA-3) (1.48 g, 85.7% yield).

Upon HPLC analysis of the obtained crystalline powder, it was found that the HPLC relative area percentage of DA-3 was 99.0%, and no over-reduced product (DA-3') was detected.

### <Synthesis Example 4> Synthesis of (E)-4-aminochlcone (compound DA-4)

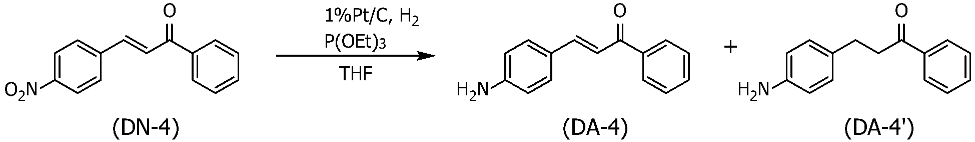

4-Nitrochlcone used herein can be a commercially available product from, for example, Tokyo Chemical Industry Co., Ltd.

### (Synthesis Example 4)

To a 200-mL pressure vessel were added 4-nitrochlcone (2.00 g, 7.90 mmol), THF (20.0 g), triethyl phosphite (0.200 g, 1.20 mmol), and 1 wt% Pt/C catalyst poisoned with 0.2 wt% iron (55.0 wt% water-containing type, 0.444 g). The resulting mixture was stirred at 40°C for 3 hours under a hydrogen atmosphere at a gauge pressure of 0.30 MPa.

Upon HPLC analysis of the reaction mixture, it was found that the HPLC relative area percentage of DA-4 was 99.7%, the reaction yield was 100%, and no over-reduced product (DA-4') was detected.

After completion of the reaction, the catalyst was removed by filtration, and the residue on the filter was washed twice with THF (2.00 g each). Subsequently, the filtrate was concentrated to dryness. To the resulting crystalline solid was added toluene (10.0 g). The mixture was stirred at 20°C for 1 hour. The crystalline solid was then collected by filtration, washed twice with toluene (2.00 g each) and dried under reduced pressure at 40°C, thereby obtaining a crystalline powder (DA-4) (1.48 g, 84.2% yield).

Upon HPLC analysis of the obtained crystalline powder, it was found that the HPLC relative area percentage of DA-4 was 97.3%, and no over-reduced product (DA-4') was detected.

### <Synthesis Example 5> Synthesis of (E)-4-aminobenzonitrile (compound DA-5)

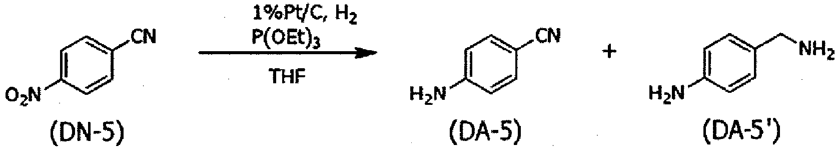

4-Nitrobenzonitrile used herein can be a commercially available product (for example, from Tokyo Chemical Industry Co., Ltd.).

### (Synthesis Example 5)

To a 200-mL pressure vessel were added 4-nitrobenzonitrile (2.00 g, 13.5 mmol), THF (20.0 g), triethyl phosphite (0.200 g, 1.20 mmol), and 1 wt% Pt/C catalyst poisoned with 0.2 wt% iron (55.0 wt% water-containing type, 0.444 g). The resulting mixture was stirred at 40°C for 12 hours under a hydrogen atmosphere at a gauge pressure of 0.30 MPa.

Upon HPLC analysis of the reaction mixture, it was found that the HPLC relative area percentage of DA-5 was 99.5%, the reaction yield was 96%, and no over-reduced product (DA-5') was detected.

### (Synthesis Example 6) Synthesis of 2,2-dimethyl-6-acetylamino-2H-1-benzopyran (compound DA-6-Ac)

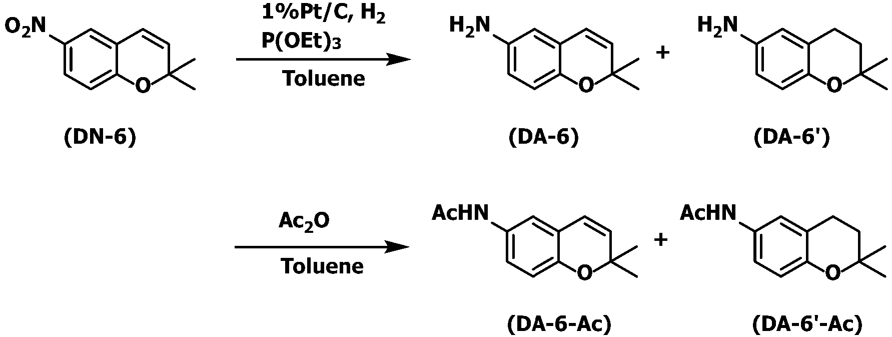

2,2-Dimethyl-6-nitro-2H-1-benzopyran (DN-6) used herein can be obtained by reaction in accordance with Japanese Patent No. 4258658.

To a 200-mL pressure vessel were added 2,2-dimethyl-6-nitro-2H-1-benzopyran (4.00 g, 19.5 mmol), toluene (40.0 g), triethyl phosphite (0791 g, 4.76 mmol), and 1 wt% Pt/C catalyst poisoned with 0.2 wt% iron (55.0 wt% water-containing type, 0.889 g). The resulting mixture was stirred at 40°C for 3 hours under a hydrogen atmosphere at a gauge pressure of 0.30 MPa.

HPLC analysis was performed in the same manner as in Synthesis Example 1, except that the eluent was changed to (A) acetonitrile/(B) 10 mM ammonium acetate (A/B = 50/50, 0 to 30 min.).

Upon HPLC analysis of the reaction mixture, it was found that the HPLC relative area percentage of DA-6 was 99.7%, and no over-reduced product (DA-6') was detected.

After completion of the reaction, the catalyst was removed by filtration, and the residue on the filter was washed twice with toluene (4.00 g each). Acetic anhydride (2.09 g, 20.5 mmol) was then added dropwise to the filtrate at 25°C over 6 minutes. The resulting mixture was stirred at 25°C for 1 hour. To the mixture was added a 4% aqueous solution of sodium hydrogen carbonate (40 g), the mixture was stirred, and the aqueous layer was separated. This operation was repeated twice. The organic layer was then concentrated to 16.8 g and cooled to 0°C. The crystalline precipitate was collected by filtration, washed twice with toluene (4.00 g each), and dried under reduced pressure at 40°C, thereby obtaining a crystalline powder (DA-6-Ac) (3.21 g, 75.8% yield).

Upon HPLC analysis of the obtained crystalline powder, it was found that the HPLC relative area percentage of DA-6-Ac was 99.6%, and no over-reduced product (DA-6'-Ac) was detected.

### (Synthesis Example 7)

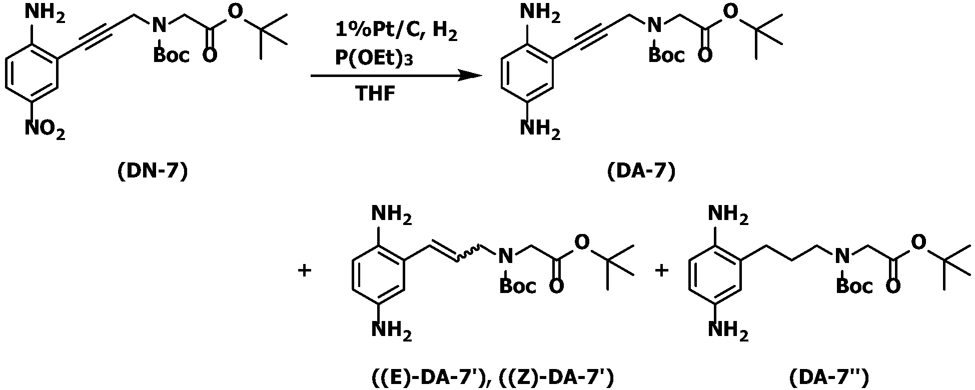

A compound (DN-7) used herein can be obtained by reaction in accordance with Japanese Patent No. 5737291.

To a 200-mL pressure vessel were added compound DN-7 (3.00 g, 7.40 mmol), THF (30.0 g), triethyl phosphite (0.600 g, 3.61 mmol), and 1 wt% Pt/C catalyst poisoned with 0.2 wt% iron (55.0 wt% water-containing type, 0.667 g). The resulting mixture was stirred at 40°C for 6 hours under a hydrogen atmosphere at a gauge pressure of 0.30 MPa.

HPLC analysis was performed in the same manner as in Synthesis Example 1, except that the eluent was changed to (A) acetonitrile/(B) 10 mM ammonium acetate (A/B = 60/40, 0 to 30 min.).

Upon HPLC analysis of the reaction mixture, it was found that the HPLC relative area percentage of DA-7 was 99.9%, and the HPLC relative area percentage of the over-reduced product ((E)-DA-7' or (Z)-DA-7', or DA-7") was 0.1%.

After completion of the reaction, the catalyst was removed by filtration, and the residue on the filter was washed twice with THF (3.00 g each). Toluene was removed from the filtrate by distillation at 50°C, to provide 13.6 g of a solution. A 12.1 g portion of this solution was dried under reduced pressure at 100°C, thereby obtaining a yellow glassy solid (DA-7) (2.45 g, 98.9% yield).

¹H-NMR (CDCl₃): δ 6.66 (s, 1H, Ar), 6.57 (s, 2H, Ar), 4.45-4.37 (m, 2H, CH₂), 4.06-3.97 (m, 2H, CH₂), 3.40 (br, 4H, 2NH₂), 3.59 (br, 4H, NH₂), 1.50-1.39 (m, 18H, 2*t-*Bu).

The entire disclosures of Japanese Patent Application No. 2023-202678 filed on November 30, 2023 and Japanese Patent Application No. 2024-029919 filed on February 29, 2024, including specifications, claims and summaries, are incorporated herein by reference in their entireties.

## Claims

1. A method for producing an aromatic amino compound, comprising: catalytic reduction of an aromatic nitro compound having, in a moiety other than an aromatic ring thereof, at least one multiple bond selected from the group consisting of a carbon-carbon bond, a carbon-nitrogen bond, and a carbon-oxygen bond, over a Pt/C catalyst in the presence of at least one phosphorus compound (A) selected from the group consisting of a phosphorus compound represented by the following formula (1), a phosphorus compound represented by the following formula (2), and a phosphorus compound represented by the following formula (3):
P(R¹)₃ (1)
where a plurality of R¹ each independently represent a hydrogen atom, a C₁₋₁₈ alkyl group, a C₁₋₁₈ alkenyl group, a C₁₋₁₈ alkoxy group, a C₆₋₂₄ aryl group, a C₆₋₂₄ aryloxy group, a C₃₋₁₈ cycloalkyl group, or a C₃₋₁₈ cycloalkoxy group, each of which may be substituted;
PO(R²)₃ (2)
where a plurality of R² each independently represent a hydrogen atom, a C₁₋₁₈ alkyl group, a C₁₋₁₈ alkenyl group, a C₁₋₁₈ alkoxy group, a C₆₋₂₄ aryl group, a C₆₋₂₄ aryloxy group, a C₃₋₁₈ cycloalkyl group, or a C₃₋₁₈ cycloalkoxy group, each of which may be substituted; and
(R³)₂P-R⁴-P(R³)₂ (3)
where a plurality of R³ each independently represents a C₁₋₁₈ alkyl group, a C₁₋₁₈ alkenyl group, a C₁₋₁₈ alkoxy group, a C₆₋₂₄ aryl group, a C₆₋₂₄ aryloxy group, a C₃₋₁₈ cycloalkyl group, or a C₃₋₁₈ cycloalkoxy group, each of which may be substituted; and R⁴ represents a C₁₋₁₈ alkylene group, a C₁₋₁₈ alkenylene group, a C₁₋₁₈ alkylenedioxy group, a C₆₋₂₄ arylene group, a C₆₋₂₄ arylenedioxy group, a C₃₋₁₈ cycloalkylene group, or a C₃₋₁₈ cycloalkylenedioxy group, each of which may be substituted.

2. The method for producing an aromatic amino compound according to Claim 1, wherein the phosphorus compound (A) is P(OMe)₃ or P(OEt)₃.

3. The method for producing an aromatic amino compound according to Claim 1, wherein the phosphorus compound (A) is used in an amount of 5 to 50 mol% relative to the aromatic nitro compound.

4. The method for producing an aromatic amino compound according to any one of Claims 1 to 3, wherein the aromatic nitro compound has any one of structures represented by the following formulas (1) to (3): 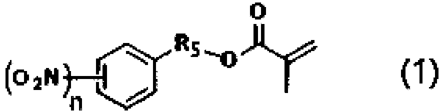 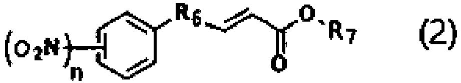 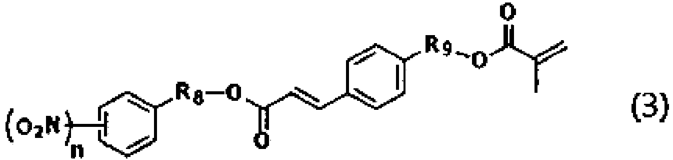 where R₅, R₆, R₈, and R₉ each independently represent a single bond or a divalent group; R₇ represents a hydrogen atom or a monovalent group; and n represents an integer of 1 or 2.

5. The method for producing an aromatic amino compound according to any one of Claims 1 to 3, wherein the Pt/C catalyst comprises 0.5 to 5 wt% platinum based on the total weight of the Pt/C catalyst.

6. The method for producing an aromatic amino compound according to any one of Claims 1 to 3, wherein the Pt/C catalyst is poisoned with iron.

7. The method for producing an aromatic amino compound according to any one of Claims 1 to 3, wherein the Pt/C catalyst is used in an amount of 1 to 20 wt% relative to the aromatic nitro compound.

8. The method for producing an aromatic amino compound according to any one of Claims 1 to 3, wherein the catalytic reduction is carried out at a reaction temperature of 0 to 100°C for a reaction time of 0.5 to 20 hours.

9. The method for producing an aromatic amino compound according to any one of Claims 1 to 3, wherein the catalytic reduction is carried out in the further presence of a vanadium compound.

10. The method for producing an aromatic amino compound according to Claim 9, wherein the vanadium compound is VO(acac)₂.

11. The method for producing an aromatic amino compound according to Claim 9, wherein the vanadium compound is used in an amount of 0.1 to 1.0 mol% relative to the aromatic nitro compound.
